# EUROPEAN PATENT APPLICATION

(11) **EP 3 026 121 A1**
(43) Date of publication of application: **01.06.2016**
(21) Application number: 14195210.1
(22) Date of filing: 27.11.2014
(51) Int. Cl.: C12Q 1/68

(54) **Micro-RNA-based diagnosis of tuberculosis**

(71) Applicant: Fraunhofer-Gesellschaft zur Förderung der angewandten Forschung e.V., 80686 München (DE)
(72) Inventor: Meyerhans, Andreas, 08013 Barcelona (ES); Latorre, Irene, 08960 Sant Just (ES); Meese, Eckart, 66882 Hütschenhausen (DE); Keller, Andreas, 66346 Püttlingen (DE); Leidinger, Petra, 66687 Wadern-Nunkirchen (DE); Backes, Christina, 66125 Saarbrücken (DE); Von Briesen, Hagen, 65110 Hünstetten (DE)
(74) Representative: Krauss, Jan

(57) **Abstract**

The present invention pertains to diagnostic methods for the detection of tuberculosis infection based on the differential expression of micro RNAs (miR) in the blood of a subject to be diagnosed. The invention further provides methods that allow for a differentiation of latent tuberculosis infection (LTBI) from active tuberculosis, based on the differential expression of a set of miR in the blood of subjects to be diagnosed. The invention provides also kits for performing the diagnostic methods as described.

SEQUENCE LISTING

<110>  Fraunhofer-Gesellschaft zur Förderung der angewandten Forschung e.V.

<120>  MICRO-RNA-BASED DIAGNOSIS OF TUBERCULOSIS

<130>  F30767EP

<160>  56

<170>  PatentIn version 3.5

<210>  1
<211>  22
<212>  RNA
<213>  Homo sapiens

<400>  1
aaugcaccug ggcaaggauu ca                                              22


<210>  2
<211>  22
<212>  RNA
<213>  Homo sapiens

<400>  2
acugcugagc uagcacuucc cg                                              22


<210>  3
<211>  23
<212>  RNA
<213>  Homo sapiens

<400>  3
agcucggucu gaggccccuc agu                                             23


<210>  4
<211>  21
<212>  RNA
<213>  Homo sapiens

<400>  4
aggggugcua ucugugauug a                                               21


<210>  5
<211>  21
<212>  RNA
<213>  Homo sapiens

<400>  5
augaccuaug aauugacaga c                                               21


<210>  6
<211>  22
<212>  RNA
<213>  Homo sapiens

<400>  6

cagugcaaug uuaaaagggc au                                              22

<210>  7
<211>  21
<212>  RNA
<213>  Homo sapiens

<400>  7
cauaaaguag aaagcacuac u                                               21

<210>  8
<211>  22
<212>  RNA
<213>  Homo sapiens

<400>  8
caugccuuga guguaggacc gu                                              22

<210>  9
<211>  23
<212>  RNA
<213>  Homo sapiens

<400>  9
   cgcauccccu agggcauugg ugu                                              23

<210>  10
<211>  22
<212>  RNA
<213>  Homo sapiens

<400>  10
cgucaacacu ugcugguuuc cu                                              22

<210>  11
<211>  22
<212>  RNA
<213>  Homo sapiens

<400>  11
cuauacaauc uauugccuuc cc                                              22

<210>  12
<211>  21
<212>  RNA
<213>  Homo sapiens

<400>  12
cugaccuaug aauugacagc c                                               21

<210>  13
<211>  22
<212>  RNA
<213>  Homo sapiens

<400>  13

cugugcgugu gacagcggcu ga                                              22

<210>  14
<211>  17
<212>  RNA
<213>  Homo sapiens

<400>  14
   gugggggaga ggcuguc                                                     17

<210>  15
<211>  22
<212>  RNA
<213>  Homo sapiens

<400>  15
guucucccaa cguaagccca gc                                              22

<210>  16
<211>  21
<212>  RNA
<213>  Homo sapiens

<400>  16
uaccacaggg uagaaccacg g                                               21

<210>  17
<211>  22
<212>  RNA
<213>  Homo sapiens

<400>  17
uagcaccauu ugaaaucggu ua                                              22

<210>  18
<211>  22
<212>  RNA
<213>  Homo sapiens

<400>  18
uagcagcaca ucaugguuua ca                                              22

<210>  19
<211>  22
<212>  RNA
<213>  Homo sapiens

<400>  19
uagcuuauca gacugauguu ga                                              22

<210>  20
<211>  23
<212>  RNA
<213>  Homo sapiens

<400>  20

ucccccaggu gugauucuga uuu                                              23

<210>  21
<211>  22
<212>  RNA
<213>  Homo sapiens

<400>  21
ucucccaacc cuuguaccag ug                                               22

<210>  22
<211>  22
<212>  RNA
<213>  Homo sapiens

<400>  22
ugagguagua guuugugcug uu                                               22

<210>  23
<211>  21
<212>  RNA
<213>  Homo sapiens

<400>  23
   uggguuuacg uugggagaac u                                                 21

<210>  24
<211>  22
<212>  RNA
<213>  Homo sapiens

<400>  24
uguaacagca acuccaugug ga                                               22

<210>  25
<211>  22
<212>  RNA
<213>  Homo sapiens

<400>  25
ugucaguuug ucaaauaccc ca                                               22

<210>  26
<211>  22
<212>  RNA
<213>  Homo sapiens

<400>  26
uuauaauaca accugauaag ug                                               22

<210>  27
<211>  19
<212>  RNA
<213>  Homo sapiens

<400>  27

aaaagcuggg uugagagga                                                    19


<210>  28
<211>  22
<212>  RNA
<213>  Homo sapiens

<400>  28
aaaagcuggg uugagagggc aa                                                 22


<210>  29
<211>  22
<212>  RNA
<213>  Homo sapiens

<400>  29
aaaagcuggg uugagagggc ga                                                 22


<210>  30
<211>  20
<212>  RNA
<213>  Homo sapiens

<400>  30
aaaagcuggg uugagagggu                                                    20


<210>  31
<211>  18
<212>  RNA
<213>  Homo sapiens

<400>  31
aaagcugggu ugagaagg                                                      18


<210>  32
<211>  22
<212>  RNA
<213>  Homo sapiens

<400>  32
acuccagccc cacagccuca gc                                                 22


<210>  33
<211>  22
<212>  RNA
<213>  Homo sapiens

<400>  33
acugcaguga aggcacuugu ag                                                 22


<210>  34
<211>  23
<212>  RNA
<213>  Homo sapiens

<400>  34

agcagcauug uacagggcua uga                                                23

<210>  35
<211>  21
<212>  RNA
<213>  Homo sapiens

<400>  35
agcuacaucu ggcuacuggg u                                                  21

<210>  36
<211>  23
<212>  RNA
<213>  Homo sapiens

<400>  36
caaagugcuc auagugcagg uag                                                23

<210>  37
<211>  23
<212>  RNA
<213>  Homo sapiens

<400>  37
caaagugcug uucgugcagg uag                                                23

<210>  38
<211>  23
<212>  RNA
<213>  Homo sapiens

<400>  38
caaagugcuu acagugcagg uag                                                23

<210>  39
<211>  22
<212>  RNA
<213>  Homo sapiens

<400>  39
cacgcucaug cacacaccca ca                                                 22

<210>  40
<211>  18
<212>  RNA
<213>  Homo sapiens

<400>  40
cagccccaca gccucaga                                                      18

<210>  41
<211>  21
<212>  RNA
<213>  Homo sapiens

<400>  41

gccccugggc cuauccuaga a                                                21

<210>  42
<211>  22
<212>  RNA
<213>  Homo sapiens

<400>  42
guggaguccu ggggaaugga ga                                               22

<210>  43
<211>  16
<212>  RNA
<213>  Homo sapiens

<400>  43
aaaggcgaca ggcaga                                                      16

<210>  44
<211>  23
<212>  RNA
<213>  Homo sapiens

<400>  44
uaaagugcuu auagugcagg uag                                              23

<210>  45
<211>  23
<212>  RNA
<213>  Homo sapiens

<400>  45
uaaggugcau cuagugcaga uag                                              23

<210>  46
<211>  23
<212>  RNA
<213>  Homo sapiens

<400>  46
uaaggugcau cuagugcagu uag                                              23

<210>  47
<211>  22
<212>  RNA
<213>  Homo sapiens

<400>  47
uagcagcaca uaaugguuug ug                                               22

<210>  48
<211>  23
<212>  RNA
<213>  Homo sapiens

<400>  48

uagugcaaua uugcuuauag ggu                                              23

<210>  49
<211>  22
<212>  RNA
<213>  Homo sapiens

<400>  49
uauggcacug guagaauuca cu                                               22

<210>  50
<211>  22
<212>  RNA
<213>  Homo sapiens

<400>  50
ucaggcucag uccccucccg au                                               22

<210>  51
<211>  22
<212>  RNA
<213>  Homo sapiens

<400>  51
ucagugcacu acagaacuuu gu                                               22

<210>  52
<211>  22
<212>  RNA
<213>  Homo sapiens

<400>  52
ucuucucugu uuuggccaug ug                                               22

<210>  53
<211>  22
<212>  RNA
<213>  Homo sapiens

<400>  53
uguaaacauc cuugacugga ag                                               22

<210>  54
<211>  22
<212>  RNA
<213>  Homo sapiens

<400>  54
uucaccaccu ucuccaccca gc                                               22

<210>  55
<211>  23
<212>  RNA
<213>  Homo sapiens

<400>  55

uuuggcacua gcacauuuuu gcu                                                23

<210>  56
<211>  22
<212>  RNA
<213>  Homo sapiens

<400>  56
uuuucaacuc uaaugggaga ga                                                 22

## Description

### FIELD OF THE INVENTION

The present invention pertains to diagnostic methods for the detection of tuberculosis infection based on the differential expression of micro RNAs (miR or miRNA) in the blood of a subject to be diagnosed. The invention further provides methods that allow for a differentiation of latent tuberculosis infection (LTBI) from active tuberculosis, based on the differential expression of a set of miR in the blood of subjects to be diagnosed. The invention provides also kits for performing the diagnostic methods as described.

### DESCRIPTION

*Mycobacterium tuberculosis.* (MTB) is a major microbial pathogen that threatens global health. The WHO has estimated about 8.6 million new tuberculosis (TB) cases and around 1.3 millions deaths due to TB in the year 2012. In MTB-infected individuals, bacteria may exist in a quiescent state for prolonged periods of time or may ultimately start multiplying and escape immune control, resulting in clinically active TB in up to 10% of infected cases. However, factors that promote progression from latent tuberculosis infection (LTBI) to disease are not completely understood.

One essential aspect for controlling the spread of TB is the ability to diagnose it in an early stage and to supply the adequate treatment. However, the commonly used test systems are still insufficient. For example, TB diagnosis by microscopy or tuberculin skin-testing (TST) has a variable and low sensitivity ranging from 20% to 60% for microscopy, and 67% to 80% for skin-testing, respectively. Tests based on MTB cultivation, the current gold standard of TB diagnosis, can take several weeks, requiring incubation periods of up to two months. Moreover, the more recent interferon-gamma release assays (IGRAS) are not able to discriminate between active TB vs. LTBI. Host biomarkers are therefore needed to diagnose TB. The ideal TB biomarker should provide prognostic information of pathogenesis and level of treatment success. Today, owing to the limited knowledge of promising TB biomarkers, global "omics" approaches are attractive options to follow.

A patient lymphocyte based test system for active tuberculosis infections is disclosed in WO 2012/164088. Via contacting a patient's lymphocytes with lipolytic mycobacterial proteins, it is possible to detect activated lymphocytes which are present only in patients having an active tuberculosis infection.

Based on the general transcriptional profile of expressed RNAs in tuberculosis patients compared to healthy controls, the disclosure of EP 2 524 966 describes a diagnostic assay using micro array analysis for assessing the presence of infections. A classifier according to the abundance of RNAs was then established as a tool for tuberculosis diagnosis.

MiRNAs are small non-coding RNAs (17-24 nucleotides in length) that regulate post-transcriptional gene expression by targeting the 3' untranslated region of messenger RNA (mRNA). They have determinant roles controlling a wide range of biological functions as cell proliferation and differentiation, metabolism and apoptosis. Recent data demonstrate that miRNAs also modulate innate and adaptive immune responses against intracellular pathogens. In addition, miRNAs can circulate in a cell-free stable form associated with microvesicles or protein complexes in body fluids like serum, plasma, saliva, urine, among others. These findings open the attractive possibility of using miRNAs as TB biomarkers for diagnosis and prognosis, and thereby avoiding time-consuming culture techniques or more invasive procedures like brocheolar lavage analysis.

In view of the state of the art procedures for diagnosing tuberculosis infections, it is an object of the present invention to provide better means for diagnosing an infection, and in particular means that allow for distinguishing a latent TB infection (LTBI) from active tuberculosis.

The above problem is solved in a first aspect a method for the diagnosis of tuberculosis in a subject, the method comprising the steps of:
(a) Providing a biological sample from the subject,
(b) Analyzing in the biological sample the expression level of at least one micro RNA (miR) selected from the group:
   (i) miR-194, miR-223, miR-374a, miR-215, miR-142-5p, miR-29c, miR-192, miR-140-3p, miR-150, miR-361-3p, miR-629, miR-342-5p, miR-15b, miR-130a, miR-210, miR-502-3p, miR-324-5p, miR-93*, let-7i, miR-532-5p, miR-629*,
   (ii) miR-21, let-7f-1*, miR-423-3p, miR-1275, miR-505; and/or
   (iii) miR-96, miR-18a, miR-20a, miR-20b, miR-93, miR-17, miR-18b, miR-15a, miR-454, miR-183, miR-106b, miR-222, miR-30e, miR-148a, miR-17*, miR-103, miR-320b, miR-484, miR-197, miR-320e, miR-3198, miR-320d, miR-320c, miR-942, miR-1305, miR-4323, miR-574-3p, miR-766, miR-320a, miR-331-3p,
(c) Comparing the expression level of the at least one miR as analysed in (b) with the expression level of the at least one miR in a control,
wherein a differential expression of the at least one miR in the biological sample compared to the control indicates the presence of a tuberculosis infection in the subject.

In preferred embodiments the above denoted miRNAs refer to micro RNA molecules having a mature miR nucleotide sequence according to SEQ ID NO: 1 to 56 in accordance to table 1 below.

**Table 1: Micro RNA Sequences**

| **Micro RNA** | **Sequence of Mature miRNA** | **SEQ ID NO:** |
|---|---|---|
| miR-502-3p | AAUGCACCUGGGCAAGGAUUCA | 1 |
| miR-93-3p | ACUGCUGAGCUAGCACUUCCCG | 2 |
| miR-423-3p | AGCUCGGUCUGAGGCCCCUCAGU | 3 |
| miR-342-5p | AGGGGUGCUAUCUGUGAUUGA | 4 |
| miR-215-5p | AUGACCUAUGAAUUGACAGAC | 5 |
| miR-130a-3p | CAGUGCAAUGUUAAAAGGGCAU | 6 |
| miR-142-5p | CAUAAAGUAGAAAGCACUACU | 7 |
| miR-532-5p | CAUGCCUUGAGUGUAGGACCGU | 8 |
| miR-324-5p | CGCAUCCCCUAGGGCAUUGGUGU | 9 |
| miR-505-3p | CGUCAACACUUGCUGGUUUCCU | 10 |
| let-7f-1-3p | CUAUACAAUCUAUUGCCUUCCC | 11 |
| miR-192-5p | CUGACCUAUGAAUUGACAGCC | 12 |
| miR-210-3p | CUGUGCGUGUGACAGCGGCUGA | 13 |
| miR-1275 | GUGGGGGAGAGGCUGUC | 14 |
| miR-629-3p | GUUCUCCCAACGUAAGCCCAGC | 15 |
| miR-140-3p | UACCACAGGGUAGAACCACGG | 16 |
| miR-29c-3p | UAGCACCAUUUGAAAUCGGUUA | 17 |
| miR-15b-5p | UAGCAGCACAUCAUGGUUUACA | 18 |
| miR-21-5p | UAGCUUAUCAGACUGAUGUUGA | 19 |
| miR-361-3p | UCCCCCAGGUGUGAUUCUGAUUU | 20 |
| miR-150-5p | UCUCCCAACCCUUGUACCAGUG | 21 |
| let-7i-5p | UGAGGUAGUAGUUUGUGCUGUU | 22 |
| miR-629-5p | UGGGUUUACGUUGGGAGAACU | 23 |
| miR-194-5p | UGUAACAGCAACUCCAUGUGGA | 24 |
| miR-223-3p | UGUCAGUUUGUCAAAUACCCCA | 25 |
| miR-374a-5p | UUAUAAUACAACCUGAUAAGUG | 26 |
| miR-320d | AAAAGCUGGGUUGAGAGGA | 27 |
| miR-320b | AAAAGCUGGGUUGAGAGGGCAA | 28 |
| miR-320a | AAAAGCUGGGUUGAGAGGGCGA | 29 |
| miR-320c | AAAAGCUGGGUUGAGAGGGU | 30 |
| miR-320e | AAAGCUGGGUUGAGAAGG | 31 |
| miR-766-3p | ACUCCAGCCCCACAGCCUCAGC | 32 |
| miR-17-3p | ACUGCAGUGAAGGCACUUGUAG | 33 |
| miR-103a-3p | AGCAGCAUUGUACAGGGCUAUGA | 34 |
| miR-222-3p | AGCUACAUCUGGCUACUGGGU | 35 |
| miR-20b-5p | CAAAGUGCUCAUAGUGCAGGUAG | 36 |
| miR-93-5p | CAAAGUGCUGUUCGUGCAGGUAG | 37 |
| miR-17-5p | CAAAGUGCUUACAGUGCAGGUAG | 38 |
| miR-574-3p | CACGCUCAUGCACACACCCACA | 39 |
| miR-4323 | CAGCCCCACAGCCUCAGA | 40 |
| miR-331-3p | GCCCCUGGGCCUAUCCUAGAA | 41 |
| miR-3198 | GUGGAGUCCUGGGGAAUGGAGA | 42 |
| miR-106b-5p | UAAAGUGCUGACAGUGCAGAU | 43 |
| miR-20a-5p | UAAAGUGCUUAUAGUGCAGGUAG | 44 |
| miR-18a-5p | UAAGGUGCAUCUAGUGCAGAUAG | 45 |
| miR-18b-5p | UAAGGUGCAUCUAGUGCAGUUAG | 46 |
| miR-15a-5p | UAGCAGCACAUAAUGGUUUGUG | 47 |
| miR-454-3p | UAGUGCAAUAUUGCUUAUAGGGU | 48 |
| miR-183-5p | UAUGGCACUGGUAGAAUUCACU | 49 |
| miR-484 | UCAGGCUCAGUCCCCUCCCGAU | 50 |
| miR-148a-3p | UCAGUGCACUACAGAACUUUGU | 51 |
| miR-942-5p | UCUUCUCUGUUUUGGCCAUGUG | 52 |
| miR-30e-5p | UGUAAACAUCCUUGACUGGAAG | 53 |
| miR-197-3p | UUCACCACCUUCUCCACCCAGC | 54 |
| miR-96-5p | UUUGGCACUAGCACAUUUUUGCU | 55 |
| miR-1305 | UUUUCAACUCUAAUGGGAGAGA | 56 |

In order to solve the aforementioned problem, the inventors have analysed the expression profile of micro RNAs (miR) in patients having active TB infection or LTBI, compared to healthy individuals. The underlying concept of this approach is that as well as for the expression of protein coding RNAs, also micro RNA expression might change in response to the bacterial infection and therefore provide expression patterns that correlate with the disease. Surprisingly the inventors developed not only miR profiles correlating with TB but also specific miR that are expressed in active TB compared to LTBI, and *vice versa.*

The term "control" refers to a sample or value from either a healthy subject, or a subject having tuberculosis in the active or latent form of infection. A control may also be a biological sample of the subject at an earlier time point. The choice which control is used is dependent on the diagnostic purpose. In the event the general presence of a tuberculosis infection is to be diagnosed, the control may be a sample from a subject not infected with a tuberculosis agent. In cases where the purpose is to detect the presence of active tuberculosis versus a LTBI also a sample from a patient having LTBI may be used as a control. If a patient being infected with a tuberculosis agent is to be monitored for the status of the infection, for example during a treatment, the control may be a sample from the patient at an earlier time point, for example a time before a tuberculosis treatment was started. Most preferred in the context of the present invention is that the control is either a sample or a reference value, derived from control samples. In certain embodiments it is preferred that the control is a healthy control, such as a control sample from an individual not being infected with a tuberculosis agent.

The term "tuberculosis" or "tuberculosis infection", as used throughout this description, indicates an infection in which the causative agent is a *Mycobacterium,* for example, *M. tuberculosis, M. bovis,* and/or *M. africanum.* In some cases, the infection can be the result of exposure to a combination of these bacterial species. Likewise, the term "tuberculosis agent" indicates an organism capable of causing tuberculosis symptoms, typically a *Mycobacterium,* for example *M. tuberculosis, M. bovis,* and/or *M. africanum.* A preferred tuberculosis pathogen in accordance with the invention is *M. tuberculosis.* Although in general the term "tuberculosis" denotes the disease caused by an infection with a tuberculosis agent, and the term "tuberculosis infection" merely refers to the infection as such, without the presence of the typical signs of tuberculosis - as is the case for a latent tuberculosis infection - the terms in any case shall be used interchangeably.

The terms "tuberculosis" or "tuberculosis infection" comprise any forms of the disease and any state of the bacterial infection, active and latent.

The terms "active tuberculosis", "active tuberculosis infection", or "tuberculosis disease" in context of the herein disclosed invention shall refer to actual disease involving the various symptoms known for tuberculosis. In active tuberculosis the bacteria overcome the defenses of the immune system and begin to multiply, resulting in the progression from latent tuberculosis infection to tuberculosis disease. Some people develop tuberculosis disease soon after infection, while others develop tuberculosis disease later when their immune system becomes weak. Typical symptoms of tuberculosis disease in a patient may comprise a skin test or blood test result indicating tuberculosis infection, an abnormal chest x-ray, or positive sputum smear or culture, actively multiplying tuberculosis bacteria in the body, the patient feels sick and may have symptoms such as coughing, fever, and weight loss. The term "active pulmonary tuberculosis" shall in particular refer to active tuberculosis with active bacteria in the lung of a patient. In some embodiments the invention pertaining to active tuberculosis preferably pertains to active pulmonary tuberculosis.

The terms "latent tuberculosis infection (LTBI)", or "latent tuberculosis", refers to a form of infection where the bacteria are not actively multiplying. Patients with latent tuberculosis infection do not feel sick and do not have any symptoms. They are infected with the bacteria, but do not have tuberculosis disease. The only sign of the tuberculosis infection is a positive reaction to the tuberculin skin test or tuberculosis blood test.

In this disclosure, the term "biomarker" or "marker" means a substance such as a miR, or a measurement of a variable related to a disease that may serve as an indicator or predictor of that disease. Biomarkers or markers are parameters from which the presence or risk of a disease can be inferred, and in embodiments may be used determine a diagnosis or prognosis or provide a measure of a disease.

In this disclosure the terms "nucleic acid", "nucleic acid sequence", and the like mean polynucleotides, which may be gDNA, cDNA or RNA and which may be single-stranded or double-stranded. The term also includes peptide nucleic acid (PNA), or any chemically DNA-like or RNA-like material.

In this disclosure the term "micro RNA" or "miR", or "miRNA" or the equivalent, means short RNA sequences, which may be between about 15 and 30 nucleotides long (or longer or shorter, preferably 21-23 nucleotides in length) and which may be non-coding. Micro RNAs regulate gene-expression post-transcriptionally by inhibiting mRNA translation. Micro-RNAs are produced as ∼70-nt hairpin RNA precursors and processed to mature miRs by RNase III nucleases Drosha and Dicer. For historical reasons some of the first discovered miRs (in *C. elegans*) are called "let" (for "lethal"), e.g. miR-let-7a. For the present invention a miR shall refer to the sequences of the mature miR as well as of any precursors thereof.

As used herein the term "diagnosing" or "diagnosis" refers to the process of identifying a medical condition or disease, in particular tuberculosis, by its signs, symptoms, and in particular from the results of various diagnostic procedures, including e.g. detecting the expression of the nucleic acids (miR) according to at least some embodiments of the invention in a biological sample obtained from an individual. Furthermore, as used herein the term "diagnosing" or "diagnosis" encompasses screening for a disease, detecting a presence or a severity of a disease, distinguishing a disease from other diseases including those diseases that may feature one or more similar or identical symptoms, providing prognosis of a disease, monitoring disease progression or relapse, as well as assessment of treatment efficacy and/or relapse of a disease, disorder or condition, as well as selecting a therapy and/or a treatment for a disease, optimization of a given therapy for a disease, monitoring the treatment of a disease, and/or predicting the suitability of a therapy for specific patients or subpopulations or determining the appropriate dosing of a therapeutic product in patients or subpopulations. The diagnostic procedure can be performed *in vivo* or preferably *in vitro*.

"Detection" as used herein refers to detecting the presence of a component (e.g., a nucleic acid sequence such as a miR or its precursor molecules) in a sample. Detection also means detecting the absence of a component. Detection also means measuring the level of a component, either quantitatively or qualitatively. With respect to the method of the invention, detection also means identifying or diagnosing tuberculosis in a subject. "Early detection" as used herein refers to identifying or diagnosing tuberculosis in a subject at an early stage of the disease (e.g., before the disease causes symptoms).

"Differential expression" as used herein refers to qualitative or quantitative differences in the temporal and/or cellular expression patterns of RNA within and among cells and tissue. Thus, a differentially expressed RNA can qualitatively have its expression altered, including an activation or inactivation, in, e.g., normal versus disease tissue. Micro RNAs, for instance, may be turned on or turned off in a particular state, relative to another state thus permitting comparison of two or more states. A qualitatively regulated transcript or miR may exhibit an expression pattern within a state or cell type that may be detectable by standard techniques. Some transcripts will be expressed in one state or cell type, but not in both. Alternatively, the difference in expression may be quantitative, e.g., in that expression is modulated, up-regulated, resulting in an increased amount of transcript or miR, or down-regulated, resulting in a decreased amount of transcript. The degree to which expression differs needs only be large enough to quantify via standard characterization techniques such as expression arrays, quantitative reverse transcriptase PCR, northern analysis, and RNase protection.

In some embodiments, the term "level" refers to the expression level of a micro RNA according to at least some embodiments of the present invention. Typically the level of the micro RNA in a biological sample obtained from the subject is different (e.g., increased or decreased) from the level of the same micro RNA in a similar sample obtained from a healthy individual or control (examples of biological samples are described herein). Alternatively, the level of the micro RNA in a biological sample obtained from the subject is different (e.g., increased) from the level of the same micro RNA in a similar sample obtained from the same subject at an earlier time point. Alternatively, the level of the micro RNA in a biological sample obtained from the subject is different (e.g., increased or decreased) from the level of the same micro RNA in an infected tissue obtained from said subject. Typically, the expression levels of the micro RNA of the invention are independently compared to their respective control level.

The term "expression level" is used broadly to include a genomic expression profile, e.g., an expression profile of micro RNAs. Profiles may be generated by any convenient means for determining a level of a nucleic acid sequence e.g. quantitative hybridization of micro RNA, labeled micro RNA, amplified micro RNA, cDNA, etc., quantitative PCR, ELISA for quantitation, and the like, and allow the analysis of differential expression between two samples. A subject or sample, e.g., cells or collections thereof, e.g., tissues or fluids, are assayed. Samples are collected by any convenient method, as known in the art. According to some embodiments, the term "expression level" means measuring the abundance of the miRNA in the biological samples.

The plurality of micro RNAs described herein, optionally includes any sub-combination of markers (i.e., micro RNA), and/or a combination featuring at least one other marker, for example a known marker. As described herein, the plurality of markers is preferably then correlated with tuberculosis, for example a micro RNA of any one of the above groups (i) to (iii). For example, such correlating may optionally comprise determining the concentration of each of the plurality of markers, and individually comparing each marker concentration to a threshold level. Optionally, if the marker concentration is above the threshold level, the marker concentration correlates with tuberculosis. Optionally, a plurality of marker concentrations correlates with tuberculosis. Alternatively, such correlating may optionally comprise determining the concentration of each of the plurality of markers, calculating a single index value based on the concentration of each of the plurality of markers, and comparing the index value to a threshold level. Also alternatively, such correlating may optionally comprise determining a temporal change in at least one of the markers, and wherein the temporal change is used in the correlating step.

A marker panel may be analyzed in a number of fashions well known to those of skill in the art. For example, each member of a panel may be compared to a "normal" value, or a value indicating a particular outcome. A particular diagnosis/prognosis may depend upon the comparison of each marker to this value; alternatively, if only a subset of markers is outside of a normal range, this subset may be indicative of a particular diagnosis/prognosis. The skilled artisan will also understand that diagnostic markers, differential diagnostic markers, prognostic markers, time of onset markers, disease or condition differentiating markers, etc., may be combined in a single assay or device. Markers may also be commonly used for multiple purposes by, for example, applying a different threshold or a different weighting factor to the marker for the different purpose(s).

In the methods of the invention, a "significant difference" in expression levels of the plurality of micro RNAs refers, in different embodiments, to a statistically significant increase or decrease, or in other embodiments to a significant increase or decrease as recognized by a skilled artisan.

The term "control" may both refer to either a control sample or a reference value. A control sample is for example derived from a subject that is not afflicted by tuberculosis (also referred to as "healthy control"), or alternatively a subject that is in a specific stage of tuberculosis. A reference value of an expression level on the other hand correlates to the expression level of a given miR in a healthy control, or alternatively in a subject being in a specific stage of the tuberculosis.

In context of the herein described invention the terms "analyzing" and "determining" are used interchangeably and refer to the direct or indirect detection of miR concentration or absolute numbers in a sample.

In one preferred embodiment of the herein described invention, micro RNA expression is compared not between the patient's sample and a healthy control, but between patient's samples derived at different time points. A differential expression of the micro RNA markers as shown in groups (i), (ii) and (iii) can therefore indicate the development of the tuberculosis infection from active to the latent infection or *vice versa.* In this embodiment the method of the invention constitutes a diagnosis in the sense of monitoring the disease progression of a subject.

### Active Tuberculosis

In one embodiment of the diagnostic methods of the invention the differential expression of a miR in group (i) in the biological sample compared to the control indicates active tuberculosis in the subject.

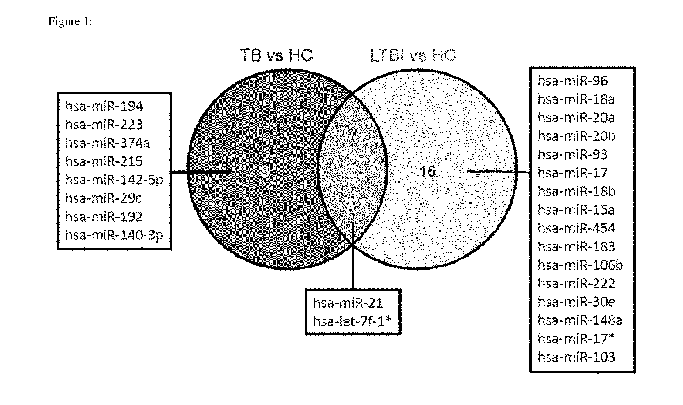

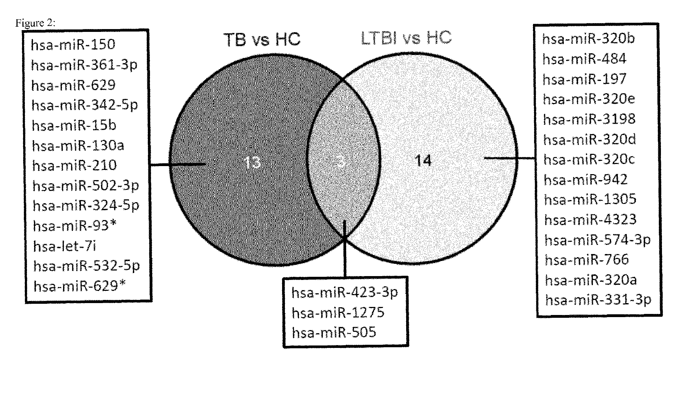

The inventors have surprisingly identified a specific set of miR that is differentially expressed only in subjects with active tuberculosis. These miR arge depicted in Fig. 1 and 2, and are selected from miR-194, miR-223, miR-374a, miR-215, miR-142-5p, miR-29c, miR-192, miR-140-3p, miR-150, miR-361-3p, miR-629, miR-342-5p, miR-15b, miR-130a, miR-210, miR-502-3p, miR-324-5p, miR-93*, let-7i, miR-532-5p, miR-629*. In this embodiment of the invention an up-regulation of any one, preferably at least two, more preferably at least three, or more, most preferably all of miRs selected from miR-194, miR-223, miR-374a, miR-215, miR-142-5p, miR-29c, miR-192, miR-140-3p compared to the control is indicative for an active tuberculosis, and preferably excludes LTBI. On the other hand a down-regulation of any one, preferably at least two, more preferably at least three, or more, most preferably all of miRs selected from miR-150, miR-361-3p, miR-629, miR-342-5p, miR-15b, miR-130a, miR-210, miR-502-3p, miR-324-5p, miR-93*, let-7i, miR-532-5p, miR-629* compared to the control is indicative for an active tuberculosis, and preferably excludes LTBI. More preferably a combination of potentially up- or down-regulated miR is analysed in a biological sample in order to diagnose active tuberculosis, or to differentiate active tuberculosis from LTBI. Active tuberculosis is in some embodiments of the invention an active pulmonary tuberculosis.

### Latent Tuberculosis Infection (LTBI)

In one further embodiment of the diagnostic methods of the invention the differential expression of a miR in group (iii) in the biological sample compared to the control indicates a latent tuberculosis infection (LTBI) in the subject.

The inventors have surprisingly identified a specific set of miR that is differentially expressed only in subjects with LTBI. These miR are depicted in Fig. 1 and 2, and are selected from miR-96, miR-18a, miR-20a, miR-20b, miR-93, miR-17, miR-18b, miR-15a, miR-454, miR-183, miR-106b, miR-222, miR-30e, miR-148a, miR-17*, miR-103, miR-320b, miR-484, miR-197, miR-320e, miR-3198, miR-320d, miR-320c, miR-942, miR-1305, miR-4323, miR-574-3p, miR-766, miR-320a, miR-331-3p. In this embodiment of the invention, an up-regulation of any one, preferably at least two, more preferably at least three, or more, most preferably all of miRs selected from miR-96, miR-18a, miR-20a, miR-20b, miR-93, miR-17, miR-18b, miR-15a, miR-454, miR-183, miR-106b, miR-222, miR-30e, miR-148a, miR-17*, miR-103 in the biological sample compared to the control is indicative for the presence of a LTBI, and preferably is indicative for a LTBI and excludes an active tuberculosis. On the other hand a down-regulation of any one, preferably at least two, more preferably at least three, or more, most preferably all of miRs selected from miR-320b, miR-484, miR-197, miR-320e, miR-3198, miR-320d, miR-320c, miR-942, miR-1305, miR-4323, miR-574-3p, miR-766, miR-320a, miR-331-3p in the biological sample compared to the control is indicative for a LTBI, and preferably is indicative for an LTBI and excludes active tuberculosis. More preferably a combination of potentially up- or down-regulated miR is analysed in a biological sample in order to diagnose LTBI, or to differentiate active tuberculosis from LTBI.

The person of skill will understand that in order to differentiate LTBI from active tuberculosis, also a combination of miR of groups (i) and (iii) may be used. Any of the miR depicted in group (ii) were differentially expressed in both LTBI and active tuberculosis. These miRs therefore are helpful in the general diagnosis of a tuberculosis infection.
Generally in context of the herein described invention a differential expression is an up-regulation or down-regulation of expression of the miR as disclosed herein in the biological sample compared to the control, preferably the control. The differential expression in group (i) is an up-regulation of miR-194, miR-223, miR-374a, miR-215, miR-142-5p, miR-29c, miR-192, miR-140-3p, and/or down-regulation of miR-150, miR-361-3p, miR-629, miR-342-5p, miR-15b, miR-130a, miR-210, miR-502-3p, miR-324-5p, miR-93*, let-7i, miR-532-5p, miR-629*; and/or in group (ii) is an up-regulation of miR-21, let-7f-1*, and/or down-regulation of miR-423-3p, miR-1275, miR-505; and/or in group (iii) is an up-regulation of miR-96, miR-18a, miR-20a, miR-20b, miR-93, miR-17, miR-18b, miR-15a, miR-454, miR-183, miR-106b, miR-222, miR-30e, miR-148a, miR-17*, miR-103, and/or down-regulation of miR-320b, miR-484, miR-197, miR-320e, miR-3198, miR-320d, miR-320c, miR-942, miR-1305, miR-4323, miR-574-3p, miR-766, miR-320a, miR-331-3p.

The diagnostic methods in accordance with the present invention are preferably *in vitro* or *ex vivo* methods.

According to some embodiments, the biological sample is selected from the group consisting of tissue, fluid or excretion samples. In another embodiments, said biological sample is selected from the group consisting of a tissue biopsy, lymph nodes, sentinel lymph nodes, blood, serum, plasma, stool, urine and peritoneal wash.

According to another embodiment, the biological sample is an excretion sample selected from urine or stool. According to a particular embodiment, the biological sample is stool. According to some embodiments, the methods of the invention are performed non-invasively.

According to another embodiment, the biological sample is a fluid sample selected from a group consisting of blood, whole blood, serum, lymph fluid, peritoneal fluid, or lavage of body cavities or organs. According to one embodiment, said biological sample is a whole blood sample. The fluid sample, in some embodiments, is aspirated from a physiological or pathological fluid. The term may also optionally encompass samples of *in vitro* cell culture constituents. The sample can optionally be diluted or eluted before performing any other diagnostic assay. Each of the above-described possibilities is a separate embodiment of the present invention.

In one embodiment, said biological sample is a tissue biopsy, preferably a lung tissue biopsy. In certain embodiments the tissue is a fresh, frozen, fixed, wax-embedded or formalin-fixed paraffin-embedded (FFPE) tissue. In particular embodiment, the tissue biopsy is a lymph node biopsy.

Most preferred is that the biological sample used in accordance with the diagnostic methods of the present invention is a blood sample, or whole blood sample, obtained from a subject suspected to suffer from tuberculosis or being infected with a *Mycobacterium,* in particular a subject suspected to have LTBI or active tuberculosis.

The term "subject" in context of the herein described invention is preferably a mammal, more preferably a human or human patient. The subject can be suspected to be healthy or having a tuberculosis infection. Alternatively the subject is already diagnosed to suffer from a tuberculosis infection, but is to be monitored with the diagnostics as provided herein.

In preferred embodiments of the invention the diagnostic methods of the invention comprise analyzing the expression level of at least two, preferably three, four, five most preferably all miR of the groups (i), (ii) and/or (iii).

Without prejudice to the above described embodiments of the invention, the inventors have also tested particular preferred combinations of miRs as disclosed herein that are particularly specific and sensitive for the diagnostic purposes of the invention. In this respect the methods of the invention in preferred embodiments provide that the miR is selected from the group consisting of miR-150, miR-18a, miR-194, miR-21, miR-223, miR-29c, miR-374a, miR-93* and miR-96. Even more preferably the miR of the invention is selected from the group consisting of miR-150, miR-21, miR-194 and miR-29c, or selected from the group consisting of miR-150, miR-21, and miR-29c. In this embodiment active tuberculosis is diagnosed in the event of a down-regulation of miR-150, and/or an up-regulation of miR-21, and/or up-regulation of miR-29c, and/or up-regulation of miR-194 in the biological sample compared to the control.

In some embodiments of the invention the following combinations of miR as disclosed herein are preferred combinations to be used in the described diagnostic methods:
(a) miR-21, miR-29c, miR-150; or
(b) miR-21, miR-29c, miR-150, miR-93*;
(c) miR-21, miR-29c, miR-150, miR-194;
(d) miR-21, miR-29c, miR-150, miR-93*, miR-194; and/or
(e) miR-21, miR-29c, miR-150, miR-93*, miR-194; miR-374a,
wherein the differential expression of all miR in any one of combinations (a) to (e) in the biological sample compared to the control indicates the presence of active tuberculosis in the subject. In one preferred embodiment of the invention the diagnostic method as described comprises analyzing the expression of a combination of miR consisting of miR-21, miR-29c, miR-150, and miR-194, wherein the differential expression of miR-21, miR-29c, miR-150, and miR-194, in the biological sample compared to the control indicates the presence of active tuberculosis in the subject.

As described herein before, the term "diagnosis" shall not only refer to the initial detection of a tuberculosis infection as such, but also refers aspects such as a monitoring of a TB patient, or wherein the diagnosis of tuberculosis in a subject is a differentiation of active tuberculosis from LTBI in the subject. The methods of the invention provide means for a continuous observation of a subject known to have LTBI to detect a switch of the infection to the active tuberculosis.

In order to determine/analyze the expression level of one or more miR according to the methods of the invention, any methodology for the quantification of miR, or quantification of miR expression that are known to the person of skill shall be comprised in the present invention. Particularly preferred methods for determining/analyzing miR expression or expression levels are from micro array, reverse transcriptase quantitative PCR (RT-qPCR), or next generation sequencing.

Most preferably the miRs as disclosed herein are human (hsa) miR sequences.

The term "differential expression,", or "differentially expressed gene" or "differentially expressed RNA" and their synonyms, which are used interchangeably, refer to a gene whose expression is activated to a higher or lower level in one physiological state relative to a second physiological subject suffering from a disease, such as tuberculosis, relative to its expression in a normal or control subject. As used herein, "gene" specifically includes nucleic acids which do not encode proteins, such as microRNAs. The terms also include miRs whose expression is activated to a higher or lower level at different states of the same disease. A differentially expressed miR may be either activated or inhibited at the mature nucleic acid level or unprocessed hairpin level. Such differences may be evidenced by a change in miR levels. Differential miR expression may include a comparison of expression between two or more miRs either as mature molecules or in preprocessed form, or a comparison of the ratios of the expression between two or more miRs or their miR products, or even a comparison of two differently processed products of the same miR, which differ between normal subjects and subjects suffering from a disease, specifically tuberculosis, or between various stages of the same disease, for example LTBI or active tuberculosis. Differential expression includes both quantitative, as well as qualitative, differences in the temporal or cellular expression pattern in a miR or its expression products among, for example, normal and diseased cells, or among cells which have undergone different disease events or disease stages. Differential miR expression is considered to be present when there is a difference of at least 1.2 fold, preferably 1.3 fold, more preferably 1.4 fold, and most preferably of at least 1.5 fold when comparing the expression level of the miR in the biological sample to the expression level in the control.

The object of the present invention is also solved by a diagnostic kit to be used in diagnosing tuberculosis, the kit comprising means of detecting a change in the expression level of one or more microRNA species associated with tuberculosis selected from the groups
(i) miR-194, miR-223, miR-374a, miR-215, miR-142-5p, miR-29c, miR-192, miR-140-3p, miR-150, miR-361-3p, miR-629, miR-342-5p, miR-15b, miR-130a, miR-210, miR-502-3p, miR-324-5p, miR-93*, let-7i, miR-532-5p, miR-629*,
(ii) miR-21, let-7f-1*, miR-423-3p, miR-1275, miR-505; and/or
(iii) miR-96, miR-18a, miR-20a, miR-20b, miR-93, miR-17, miR-18b, miR-15a, miR-454, miR-183, miR-106b, miR-222, miR-30e, miR-148a, miR-17*, miR-103, miR-320b, miR-484, miR-197, miR-320e, miR-3198, miR-320d, miR-320c, miR-942, miR-1305, miR-4323, miR-574-3p, miR-766, miR-320a, miR-331-3p.

In some embodiments, the kit further comprises means for collecting a sample (e.g., whole blood) from a subject. In another embodiment, the diagnostic kit further comprises instructions for performing the necessary steps for determining miRNAs expression levels, e.g., in a sample obtained from a subject.

Means of detecting a change in the expression level of one or more miR in accordance with the present invention are particularly any kinds of nucleic acid probes that may directly bind to a miR species of the invention. Such probes are well known to the skilled artisan and may be labeled with any markers to allow for a detection of binding to the miR species of the invention. Further preferred means include any nucleic acid Chips or micro arrays comprising probes specific for one or more of the miR of the present invention.

A preferred diagnostic kit in accordance with the present invention comprises means of detecting the level of all miR species of any one of the groups:
(a) miR-21, miR-29c, miR-150; or
(b) miR-21, miR-29c, miR-150, miR-93*;
(c) miR-21, miR-29c, miR-150, miR-194;
(d) miR-21, miR-29c, miR-150, miR-93*, miR-194; and/or
(e) miR-21, miR-29c, miR-150, miR-93*, miR-194; miR-374a.

Even more preferred is a kit according to the invention, comprising means of detecting the level of all miR species miR-21, miR-29c, miR-150, and miR-194.

The object of the present invention is then solved also by a use of a kit as described herein for the diagnosis of tuberculosis, preferably active tuberculosis, more preferably active pulmonary tuberculosis, or for use in the differentiation of active tuberculosis from LTBI. Preferably the diagnosis comprises a method as described herein.

The present invention will now be further described in the following examples with reference to the accompanying figures and sequences, nevertheless, without being limited thereto. For the purposes of the present invention, all references as cited herein are incorporated by reference in their entireties. In the Figures:
- **Figure 1:**: A set of 8 miRNAs are differentially up-regulated during active TB by microarray. Venn diagram of up-regulated miRNAs with unadjusted p-value <0.05 and deregulated with a fold change >1.3 in TB vs HC and LTBI vs HC. Overlapping miRNAs between the two comparisons are also visualized. TB: tuberculosis; HC: healthy controls; LTBI: latent tuberculosis infection.
- **Figure 2:**: A set of 13 miRNAs are differentially down-regulated during active TB by microarray. Venn diagram of down-regulated miRNAs with unadjusted p-value <0.05 and deregulated with a fold change >1.3 in TB vs HC and LTBI vs HC. Overlapping miRNAs between the two comparisons are also visualized. TB: tuberculosis; HC: healthy controls; LTBI: latent tuberculosis infection

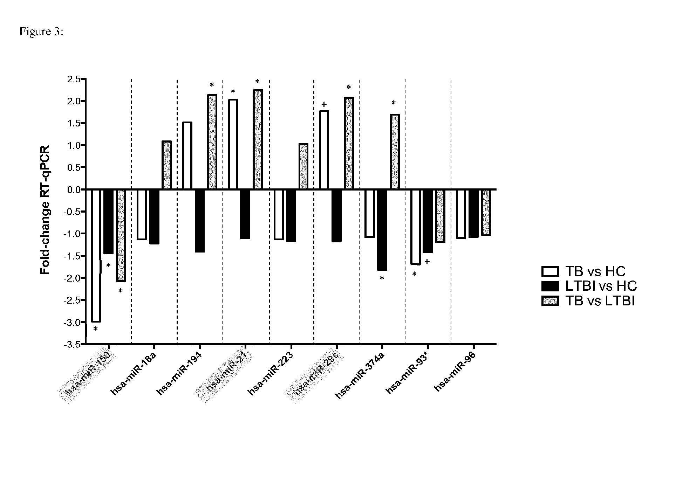

- **Figure 3:**: Relative fold-change of miRNA transcripts of whole blood from different patient groups and controls. The miRs 21, 29 and 150 are simultaneously deregulated in TB patients respect HC and LTBI individuals. Data represents the mean fold-change of each miRNA validated by real time-quantitative PCR for TB vs HC (white bars), LTBI vs HC (black bars) and TB vs LTBI (grey bars). TB: tuberculosis; HC: healthy controls; LTBI: latent tuberculosis infection; RT-qPCR: Real time-quantitative polymerase chain reaction. *p<0.05; + p=0.06 (near significance).

### EXAMPLES

### Materials and Methods

### Patients and inclusion criteria

Blood samples were collected from five different institutions located in Barcelona (Spain). Participating centres were Hospital Universitari Germans Trias i Pujol, Serveis Clinics, Unitat de Prevenció i Control de la Tuberculosi (CAP Drassanes) and Hospital del Mar. Blood was extracted using PAXgene Blood RNA tubes (PreAnalytiX). Written informed consent from all blood donors participating in the study was obtained. The corresponding Ethics Committees provided ethics approval. Demographical and clinical patient data was collected by a detailed questionnaire.

The inventors prospectively enrolled a total of 50 patients, who were classified into three groups:
- Group 1:: 17 individuals with LTBI coming from contact tracing studies. The inclusion criteria for all patients were a positive TST, at least one positive result for one of the IFN-γ assays (T-SPOT.TB and/or QFN-G-IT) and no more than two weeks of chemoprophylaxis.
- Group 2:: 17 active TB patients. The inclusion criteria were a positive culture for MTB and no more than two weeks of anti-TB therapy.
- Group 3:: 16 healthy individuals with negative TST and negative IFN-γ assay results (T-SPOT.TB and/or QFN-G-IT) who were identified by routine examinations.

The main demographic characteristics of the patients included in the study are summarized in Table 2.

### Tuberculin skin test

Two intradermal tuberculin units of PPD RT23 (Statens Serum Institute, Copenhagen, Denmark) were used to perform the TST. The tuberculin was administered according to the Spanish Society of Pneumology guidelines. A chest radiograph was taken from all individuals with positive TST indurations (≥5 mm). Chemoprophylaxis was given to asymptomatic patients with normal chest radiography while TB patients were treated with standard combination therapy.

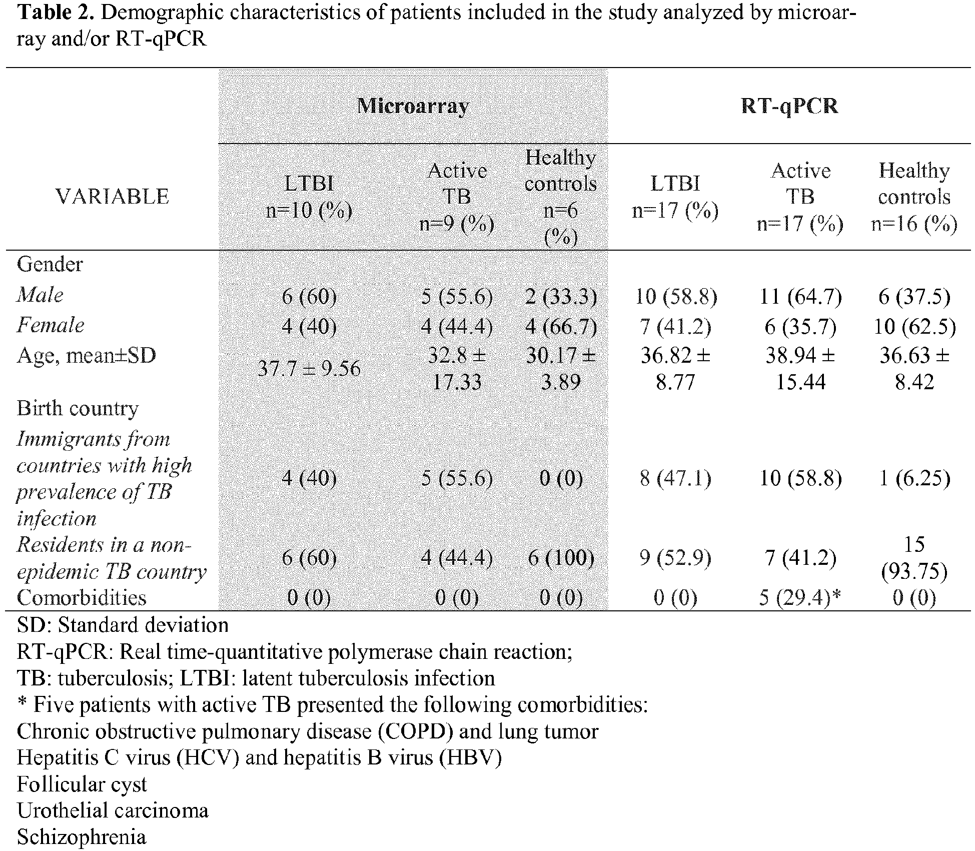

### Interferon-gamma release assays (IGRAS)

IGRAs rely on the detection of IFN-γ secreted by effector T cells after specific stimulation with MTB antigens (ESAT-6, CFP-10 and TB7.7). In this study, the inventors used the commercial QuantiFERON-TB Gold In-Tube (Qiagen, Germany) and T-SPOT.TB (Oxford Immunotec, UK) assays. IGRAs were performed and interpreted according to the manufacturer's instructions.

### RNA isolation and quality control

A total of 2.5 ml of blood was extracted in PAXgene Blood RNA tubes (PreAnalytiX) that ensure stabilization of RNA and hence stabilization of the miRNA expression profiles. The PAXgene Blood miRNA kit (Qiagen) was used to isolate total RNA (>18 nucleotides including miRNA). The protocol is from the manual of the PAXgene Blood miRNA kit. Isolated RNA was stored at -80°C until use.

Absorbance measurements at 260 and 280 nm were made on a spectrophotometer (NanoDrop 2000 Spectrophotometer, Thermo Scientific) in order to determine RNA concentration and purity. A ratio between 1.8-2.0 of absorbance at 260 nm and 280 nm corresponds to a sufficient RNA purity for subsequent microarray experiments. RNA integrity was determined with the Agilent RNA 6000 Pico kit (Agilent Technologies). Agilent's 2100 Bioanalyzer software estimated the integrity of total RNA samples (RIN). In general, a RIN value higher than 7-8 was accepted to work well for miRNA expression profiling by microarray.

### Identification of miRNA expression patterns by microarray hybridization

The inventors performed microarray analysis as previous described using SurePrint G3 8 × 60K miRNA microarrays (Agilent, Cat No. G4870A) that contained 40 replicates of each of the 1,205 miRNAs of miRBase v16 (http://www.mirbase.org). Microarray scan data were further processed using Feature Extraction software (Agilent).

### Real time-quantitative polymerase chain reaction (RT-qPCR) validation

RT-qPCR was performed in nine selected miRNAs (hsa-miR-150, hsa-miR-18a, hsa-miR-194, hsa-miR-21, hsa-miR-223, hsa-miR-29c, hsa-miR-374a, hsa-miR-93* and hsa-miR-96) for validation and confirmation of microarray results. The miScript PCR System (Qiagen) was used. All procedures were performed according manufacture's instructions and recommendations. First, using a total amount of 200 ng RNA, the inventors selectively transcribed mature miRNAs into cDNA using the miScript HiSpec Buffer and the miScript II RT Kit. Second, RT-qPCR was performed with the miScript SYBR Green PCR Kit. The inventors used the two endogenous controls, i.e., the snoRNA RNU48 (official symbol: SNORD48) and the snRNA RNU6B (official symbol: RNU6-2) for normalization of threshold cycle (Ct) values in samples. All samples were run in duplicates. Fold-change values were computed by using the 2-ΔΔCt method.

### Bioinformatic analysis

Microarray data was log-transformed and quantile normalization was applied to normalize expression values using the freely available R software. MiRNA expression values were compared between different groups of patients by employing an unpaired two-tailed t-test. The fold-change calculated for each miRNA indicated down-regulation or up-regulation when the value was negative or positive, respectively. Area under the curve (AUC), fold-change and significance (p-value) were calculated for each miRNA. AUC values suggest how well a miRNA can separate between groups. This parameter indicates good discriminatory capacity when it is close to 1 or 0 values. Differences were considered significant when the p-value was lower than 0.05. In this study, no adjusted p-values were considered. Sensitivity, specificity, accuracy and positive predictive values (PPV) were calculated for active TB diagnosis using a Support Vector machines (SVM) with linear kernel and employing 20 repetitions of standard 10-fold cross validation.

### Example 1: Analysis of blood-derived miRNA levels by microarray

To identify miRNAs that are differentially expressed between TB patients, LTBI and healthy controls, blood samples from the 3 study groups were analysed by microarrays. For this first analysis, a total of 25 samples were tested representing 9 (pulmonary) active TB patients, 10 individuals with LTBI and 6 healthy controls. They were randomly picked from all available samples excluding patients with comorbidities (Table 1). Total RNA with length greater than 18 nucleotides was isolated and subjected to microarray-based quantification covering 1,205 different miRNAs. Overall, 188 miRNAs were expressed in all active TB patients, 199 in all LTBI individuals and 216 in all healthy controls. For 827 miRNAs, no expression was observed.

An unpaired two-tailed t-test was applied to evaluate statistical differences in miRNA expression between groups. For further analysis, miRNAs were selected by the following criteria, (i) an unadjusted p-value <0.05 for the comparisons, (ii) detection in all samples of at least one group of each comparison, and (iii) up-regulation or down-regulation with a fold-change >1.3. According to these criteria, a total of 26 miRNAs were deregulated in active TB patients with respect to healthy controls (TB vs. HC): 10 miRNAs were up-regulated and 16 miRNAs were down-regulated. MiRNA levels in the active TB group compared with controls were between 3.27-1.32 fold over-expressed and 2.02-1.31 fold under-expressed, respectively (Table 3). When comparing LTBI individuals with healthy controls (LTBI vs. HC), a total of 35 miRNAs were deregulated: 18 miRNAs were up-regulated and 17 miRNAs down-regulated. Interestingly, the comparison between TB vs. HC and LTBI vs. HC groups only provided a small overlap of 5 deregulated miRNAs: hsa-miR-21, hsa-let-7f-1*, hsa-miR-423-3p, hsa-miR-1275 and hsa-miR-505 (Figures 1 and 2) indicating a good separation between the two comparisons.

**Table 3. Up-regulated and down-regulated miRNAs in TB blood with an unadjusted p-value <0.05 for the comparisons of TB versus HC with a deregulated fold-change >1.3.**

| **miRNA** | **Fold-change TB *vs*. HC** | **Deregulation** |
|---|---|---|
| hsa-miR-194 | 3.27 | Up-regulated |
| hsa-miR-223 | 2.62 | Up-regulated |
| hsa-miR-374a | 2.09 | Up-regulated |
| hsa-miR-215 | 1.73 | Up-regulated |
| hsa-miR-142-Sp | 1.63 | Up-regulated |
| hsa-miR-29c | 1.60 | Up-regulated |
| hsa-miR-21 | 1.58 | Up-regulated |
| hsa-miR-192 | 1.41 | Up-regulated |
| hsa-let-7f-1* | 1.38 | Up-regulated |
| hsa-miR-140-3p | 1.32 | Up-regulated |
| hsa-miR-150 | -2.02 | Down-regulated |
| hsa-miR-361-3p | -1.85 | Down-regulated |
| hsa-miR-1275 | -1.82 | Down-regulated |
| hsa-miR-629 | -1.75 | Down-regulated |
| hsa-miR-342-5p | -1.72 | Down-regulated |
| hsa-miR-15b | -1.71 | Down-regulated |
| hsa-miR-130a | -1.69 | Down-regulated |
| hsa-miR-423-3p | -1.64 | Down-regulated |
| hsa-miR-210 | -1.60 | Down-regulated |
| hsa-miR-502-3p | -1.55 | Down-regulated |
| hsa-miR-324-5p | -1.52 | Down-regulated |
| hsa-miR-93* | -1.47 | Down-regulated |
| hsa-let-7i | -1.42 | Down-regulated |
| hsa-miR-532-5p | -1.41 | Down-regulated |
| hsa-miR-629* | -1.37 | Down-regulated |
| hsa-miR-505 | -1.31 | Down-regulated |

| | | |
|---|---|---|
| TB: tuberculosis; HC: Healthy controls | | |

### Example 2: Validation of selected, differentially-expressed miRNAs by RT-qPCR

The following nine miRNAs were selected and further analyzed by RT-qPCR: hsa-miR-150, hsa-miR-18a, hsa-miR-194, hsa-miR-21, hsa-miR-223, hsa-miR-29c, hsa-miR-374a, hsa-miR-93* and hsa-miR-96. The selection criteria were (i) a strong fold-change deregulation and/or (ii) relevance as suggested in the literature. They were assessed with the samples from the complete study group covering 17 (pulmonary) active TB patients, 17 individuals with LTBI and 16 healthy controls. Five patients with active TB presented comorbidities. Detailed characteristics are summarized in Table 2.

Fold-change values for the three comparisons TB vs. HC, LTBI vs. HC and TB vs. LTBI were calculated for each miRNA. Three of the nine miRNAs studied were significantly deregulated in active TB patients with respect to those with LTBI and healthy controls. These were hsa-miR-150 (down-regulation), hsa-miR-21 (up-regulation) and hsa-miR-29c (up-regulation; nearly significant for TB vs. HC) with mean fold-change values higher than 1.5 (Figure 3). Furthermore, hsa-miR-194 was significantly up-regulated with a fold-change >1.5 in active TB patients with respect to LTBI; and hsa-miR-93* showed significant down-regulation in active TB patients in comparison with controls. These RT-qPCR results were not totally consistent with those obtained in the microarray-based analysis. While most of validated miRNAs showed the same direction of deregulation, in some occasions the microarray threshold of detecting expression differences seemed quite low. This enforces the need for RT-qPCR validation.

### Example 3: Diagnostic test performance for active TB

In order to define and evaluate a miRNA-signature for rapid pulmonary TB diagnosis, validated miRNAs with mean fold-change values >1.5, and a significant deregulation in the comparisons TB vs. LTBI and/or TB vs. HC were selected. These were hsa-miR-150, hsa-miR-21, hsa-miR-29c and hsa-miR-194 (Figure 3). The inventors then applied a Support Vector Machine with linear kernel (20 repetitions of standard 10-fold cross validation) for computing the accuracy, the sensitivity, the specificity and positive predictive values (PPV) with the corresponding 95% confidence intervals. Interestingly, the selected miRNA-signature had a 90.1% accuracy (89.75 - 90.45%), a 91.21% sensitivity (90.8 - 91.62%), a 87.95% specificity (87.37 - 88.52%) and a 93.63% PPV (93.35 - 93.91%) for diagnosing active TB (for comparison TB vs. LTBI+HC). The AUC value obtained was 0.068 indicating that this miRNA combination has a very good discriminatory capacity.

The development of a rapid assay for active TB diagnosis with increased sensitivity and specificity continues to be an important goal in clinical microbiology. Given the great success of recent miRNA-based diagnostics in various human pathologies [16] including bacterial infections [17], the inventors have assessed in the present study the potential of miRNA patterns as biomarker to distinguish between active TB and LTBI. Whole blood from active TB patients, LTBI individuals and healthy controls was first screened for differentially expressed miRNAs with a chip microarray. Promising deregulated miRNAs were then selected and validated by RT-qPCR. A signature of four miRNAs was identified and enabled to diagnose active TB with 91.21% sensitivity and 87.95% specificity within a single working day.

Several studies aiming to identify deregulated miRNAs in active TB have been performed [18-30], however, only some of them addressed the issue of diagnostic performance [18, 21, 22, 27, 28]. The data of these studies revealed that the quantification of a single miRNA is not a reliable indicator of active TB as it was associated with a low sensitivity [18, 21]. Amongst the reasons is the neglect of miRNA expression differences between individuals. When miRNAs were evaluated in combination, then reasonable sensitivities and specificities were reached. For example, serum-derived miR-378, miR-483-5p, miR-22, miR-29c, miR-101 and miR-320b together discriminated active TB from healthy controls with 95% sensitivity and 91.8% specificity [28]. With a different, non-overlapping set of 15 miRNAs from serum, a comparable discriminatory capacity was observed [27]. Both study results are in the order of the test performance described here using just 4 miRNAs quantified from whole blood sampling.

It is intriguing to see that so different miRNA signatures are being proposed for the diagnosis of active TB. Clearly they must be related to one or several differences in study design and experimental details. Most studies were composed of 2 separate experimental steps, a primary miRNA screen and a subsequent result validation by RT-qPCR. Therefore, the resolution of the primary screen, for example a microarray platform or RNA sequencing, as well as the patient grouping influences the choice of miRNAs to be evaluated further and thus the final miRNA signature [31]. Furthermore, even when neglecting differences in reference gene selection or measures of RNA quality control, the choice of starting material for miRNA quantification like serum, individually separated blood cell populations or whole blood is expected to affect miRNA signatures. To take miR-29a as an example, Yi and co-workers found a significant overexpression in serum and sputum of TB patients compared with healthy controls [23], while Kleinsteuber et al. observed it lower expressed in CD4+ T cells from active TB adults compared to LTBI individuals, as well as lower expressed in whole blood from children with active TB [24]. Likewise, in the present study the inventors have observed an overexpression of miR-21 in TB vs. LTBI and healthy controls while Kleinsteuber et al. found it lower expressed [24]. This is suggestive of an important impact of the patient materials on the miRNA quantification results and calls for a need of assay standardization [12].

The observed link between a deregulated miRNA signature and active TB urges to better understand the underlying mechanisms. Previous studies from others already gave a functional hint for the found 4 miRNAs. Ma and co-workers have described that miR-29 suppresses the immune response to intracellular pathogens by targeting IFN-□ mRNA [32]. Moreover, miR-29 also targets the mRNAs for Bcl-2 and Mcl-1. As these transcripts code for two central anti-apoptotic proteins, an upregulation of miR-29 may inactivate Bcl-2 and Mcl-1 mRNAs leading to an increased apoptosis of cells involved in the anti-TB response [33, 34]. Another up-regulated miRNA of this study was miR-21. It is involved in promoting the production of the anti-inflammatory cytokine IL-10 [35] while inhibiting IL-12, a key cytokine involved in Th1 cell polarization. A consequence may be the reduction of host Th1 responses [36, 37] that are an important component of MTB control. The third overexpressed miRNA in the blood of active TB patients was miR-194 for which the possible relation to TB is less obvious. However, together with miR-29 it targets components of the Wnt signaling pathway that seems to play a role in TB pathogenesis. Finally, miR-150 is under-expressed in active TB. A primary target of this miRNA is c-Myb, a negative regulator of natural killer (NK) and invariant NK T (iNKT) cell maturation. Thus a reduction of mR-150 levels may indicate the development of fewer mature NK and iNKT cells which in turn represent early innate effector cells controlling invading pathogens [40]. Altogether, these findings are in line with the hypothesis that the four deregulated miRNAs can create an immunologically favorable environment for MTB expansion and active TB development.

## Claims

1. A method for the diagnosis of tuberculosis in a subject, the method comprising the steps of:
(a) Providing a biological sample from the subject,
(b) Analyzing in the biological sample the expression level of at least one micro RNA (miR) selected from the group:
(i) miR-194, miR-223, miR-374a, miR-215, miR-142-5p, miR-29c, miR-192, miR-140-3p, miR-150, miR-361-3p, miR-629, miR-342-5p, miR-15b, miR-130a, miR-210, miR-502-3p, miR-324-5p, miR-93*, let-7i, miR-532-5p, miR-629*,
(ii) miR-21, let-7f-1*, miR-423-3p, miR-1275, miR-505; and/or
(iii) miR-96, miR-18a, miR-20a, miR-20b, miR-93, miR-17, miR-18b, miR-15a, miR-454, miR-183, miR-106b, miR-222, miR-30e, miR-148a, miR-17*, miR-103, miR-320b, miR-484, miR-197, miR-320e, miR-3198, miR-320d, miR-320c, miR-942, miR-1305, miR-4323, miR-574-3p, miR-766, miR-320a, miR-331-3p,
(c) Comparing the expression level of the at least one miR as analysed in (b) with the expression level of the at least one miR in a control,
wherein a differential expression of the at least one miR in the biological sample compared to the control indicates the presence of a tuberculosis infection in the subject.

2. The method according to claim 1, wherein a differential expression of a miR in group (i) in the biological sample compared to the control indicates active tuberculosis in the subject.

3. The method according to claim 1, wherein a differential expression of a miR in group (iii) in the biological sample compared to the control indicates a latent tuberculosis infection (LTBI) in the subject.

4. The method according to any of claims 1 to 3, which is an *in-vitro* or *ex-vivo* method.

5. The method according to any of claims 1 to 4, wherein the biological sample is selected from whole blood, serum, plasma, saliva or urine.

6. The method according to any of claims 1 to 5, wherein the miR is selected from the group consisting of miR-194, miR-150, miR-18a, miR-21, miR-223, miR-29c, miR-374a, miR-93* and miR-96.

7. The method according to any of claims 1 to 6, comprising analyzing the expression level of a miR-combination selected from:
(a) miR-21, miR-29c, miR-150;
(b) miR-21, miR-29c, miR-150, miR-93*;
(c) miR-21, miR-29c, miR-150, miR-194;
(d) miR-21, miR-29c, miR-150, miR-93*, miR-194; and/or
(e) miR-21, miR-29c, miR-150, miR-93*, miR-194; miR-374a
wherein the differential expression of all miR in any one of combinations (a) to (e) in the biological sample compared to the control indicates the presence of active tuberculosis in the subject.

8. The method according to any of claims 1 to 7, comprising analyzing the expression of a combination of miR consisting of miR-21, miR-29c, miR-150, and miR-194, wherein the differential expression of miR-21, miR-29c, miR-150, and miR-194, in the biological sample compared to the control indicates the presence of active tuberculosis in the subject.

9. The method according to any of claims 1 to 8, wherein the diagnosis of tuberculosis in a subject is a differentiation of active tuberculosis from LTBI in the subject.

10. The method according to any of claims 1 to 9, wherein analyzing the expression of a miR comprises the use of an assay selected from micro array, reverse transcriptase quantitative PCR (RT-qPCR), or next generation sequencing.

11. The method according to any of claims 1 to 10, wherein the miR is a human (hsa) miR.

12. The method according to any of claims 1 to 11, wherein a differential expression (up-regulation or down-regulation) is a difference of at least 1.2 fold, preferably 1.3 fold, more preferably 1.4 fold, and most preferably of at least 1.5 fold when comparing the expression level of the miR in the biological sample to the expression level

13. A kit to be used in diagnosing tuberculosis comprising means of detecting a change in the level of one or more microRNA species associated with tuberculosis selected from the groups
(i) miR-194, miR-223, miR-374a, miR-215, miR-142-5p, miR-29c, miR-192, miR-140-3p, miR-150, miR-361-3p, miR-629, miR-342-5p, miR-15b, miR-130a, miR-210, miR-502-3p, miR-324-5p, miR-93*, let-7i, miR-532-5p, miR-629*,
(ii) miR-21, let-7f-1*, miR-423-3p, miR-1275, miR-505; and/or
(iii) miR-96, miR-18a, miR-20a, miR-20b, miR-93, miR-17, miR-18b, miR-15a, miR-454, miR-183, miR-106b, miR-222, miR-30e, miR-148a, miR-17*, miR-103, miR-320b, miR-484, miR-197, miR-320e, miR-3198, miR-320d, miR-320c, miR-942, miR-1305, miR-4323, miR-574-3p, miR-766, miR-320a, miR-331-3p,

14. Use of a kit according to claim 13, in the diagnosis of tuberculosis, preferably active tuberculosis, or for use in the differentiation of active tuberculosis from LTBI.

15. The use according to claim 14, wherein the diagnosis comprises a method according to any of claims 1 to 12.
